# EUROPÄISCHE PATENTSCHRIFT

(11) **EP 0 757 980 B1**
(45) Veröffentlichungstag und Bekanntmachung des Hinweises auf die Patenterteilung: **29.10.1997**
(21) Anmeldenummer: 95112482.5
(22) Anmeldetag: 09.08.1995
(51) Int. Cl.: C07C 209/48, C07C 209/26, C07C 253/30, C07C 211/36

(54) **Verfahren zur Herstellung von 3-Aminomethyl-3,5,5-trimethylcyclohexylamin**
Process for the preparation of 3-aminomethyl-3,5,5-trimethyl cyclohexylamine
Procédé de préparation d'aminométhyl-3 triméthyl-3,5,5 cyclohexylamine

(43) Veröffentlichungstag der Anmeldung: 12.02.1997
(73) Patentinhaber: BASF AKTIENGESELLSCHAFT, 67056 Ludwigshafen (DE)
(72) Erfinder: Witzel, Tom, Dr., D-67069 Ludwigshafen (DE); Voit, Guido, Dr., D-69198 Schriesheim (DE)

(56) Entgegenhaltungen:
- EP-A- 0 042 119
- EP-A- 0 394 967
- EP-A- 0 449 089
- EP-A- 0 590 419
- US-A- 4 248 799

## Beschreibung

Die vorliegende Erfindung betrifft ein Verfahren zur Herstellung von 3-Aminomethyl-3,5,5-trimethylcyclohexylamin (Isophorondiamin, IPDA) durch Umsetzung von 3-Cyano-3,5,5-trimethylcyclohexanon (Isophoronnitril, IPN) mit Isophorondiamin und anschließender oder gleichzeitiger Zugabe von Ammoniak unter einem Wasserstoffdruck von 50 bis 300 bar in Gegenwart eines Hydrierkatalysators ohne Abtrennung des Reaktionswassers.

Die Herstellung von 3-Aminomethyl-3,5,5-trimethylcyclohexylamin durch reduktive Aminierung von 3-Cyano-3,5,5-trimethylcyclohexanon (erhältlich aus Cyanwasserstoff und Isophoron, z.B. DE-A-12 40 854, US-A-5 011 968) ist allgemein bekannt. Als Nebenprodukte werden vor allem 3-Aminomethyl-3,5,5-trimethylcyclohexanol (Aminoalkohol, IPAA), 1,3,3-Trimethyl-6-aza-bicyclo-(3,2,1)octan (Bicyclus), 3-Cyano-3,5,5-trimethylcyclohexylamin (Aminonitril, IPNA) und die durch Blausäureabspaltung entstehenden Spaltprodukte 3,5,5-Trimethylcyclohexanol und 3,5,5-Trimethylcyclohexylamin aufgeführt.

In DE-A-12 29 078 werden bei Einsatz von 10 bis 30 mol Ammoniak pro mol 3-Cyano-3,5,5-trimethylcyclohexanon an einem Cobalt-Katalysator (33 % auf Kieselgur) bei diskontinuierlicher Fahrweise im Rührautoklaven bei 150 bar und 120°C Ausbeuten bis zu 81,4 % angegeben. Die Reaktion wird in einem organischen Lösungsmittel durchgeführt, beispielhaft in 1 kg Methanol pro kg 3-Cyano-3,5,5-trimethylcyclohexanon. Laut DE-A-41 06 882 erwiesen sich die Ausbeuten als nicht reproduzierbar.

Im Bestreben, die Ausbeute zu erhöhen, wird in DE-A-30 21 955 die Reaktion zweistufig bei 250 bis 300 bar und einem Ammoniak-Überschuß von 10 bis 30 mol/mol durchgeführt (bis zu 95 % Ausbeute). Hierbei werden in der ersten Stufe organische oder anorganische Ionenaustauscher in der Ammoniumform eingesetzt, um eine Iminierung der Ketofunktion zu erreichen. Das aus Ammoniak und 3-Cyano-3,5,5-trimethylcyclohexanon gebildete Imin wird dann in einer zweiten Stufe durch Hydrierung an konventionellen Cobaltkatalysatoren zu 3-Amino-methyl-3,5,5-trimethylcyclohexylamin umgesetzt.

Wird wie in DE-A-30 11 656 beschrieben die Iminierung ohne Katalysator - also rein thermisch - durchgeführt, so ist sowohl die Selektivität als auch die Raum-Zeit-Ausbeute deutlich schlechter.

DE-A-40 10 227 beschreibt die 3-Amino-methyl-3,5,5-trimethylcyclo-hexylamin-Herstellung ebenfalls zweistufig, wobei die Iminierung an aciden Metalloxiden und die Hydrierung bevorzugt an basisch-dotierten Hydrierkatalysatoren durchgeführt wird. Es werden Ausbeuten zwischen 95 bis 98% erzielt.

Nach der DE-A-41 06 882 ist bei der Herstellung von 3-Aminomethyl-3,5,5-trimethylcyclohexylamin aus 3-Cyano-3,5,5-trimethylcyclohexanon unter aminierenden Hydrierbedingungen an Raney-Cobalt oder Raney-Nickel zum Erreichen hoher Selektivitäten ein zusätzlicher Cokatalysator in Form eines Salzes notwendig (beispielhaft 50 g Kobaltchlorid-hexahydrat auf 100 g Raney-Cobalt und 400 g 3-Cyano-3,5,5-trimethylcyclohexanon), wobei die Ausbeute bei 85 % liegt. Die Nachteile dieses Verfahrens liegen außerdem im Einsatz eines organischen Lösungsmittels (beispielhaft 2 kg/kg 3-Cyano-3,5,5-trimethylcyclohexanon) und in der schwierigen Wiedergewinnung des korrosiven Katalysator/Cokatalysator-Systems.

Aus der EP-A-394 967 ist ein Verfahren zur Herstellung von 3-Aminomethyl-3,5,5-trimethylcyclohexylamin bekannt, bei dem eine Reihe von Maßnahmen eingehalten werden müssen, um die Direkthydrierung der Carbonylfunktion zum Alkohol unter Bildung von 3-Aminomethyl-3,5,5-trimethylcyclohexanol und die Blausäure-Abspaltung unter Bildung von 3,5,5-Trimethylcyclohexanol und 3,5,5-Trimethylcyclohexylamin zurückzudrängen:
- zwei- und mehrstufige Fahrweise mit Druck und Temperaturstaffelung
- Zusatz von Alkoholen als Aminierungspromotoren
- Verschiebung des Iminierungsgleichgewichts durch Abtrennung des Reaktionswassers

Letztere Maßnahme ist bei der Iminierung mit Ammoniak aufgrund der Siedepunkte nicht möglich. Es wird daher der Umweg aufgezeichnet, IPN zuerst mit eihem hochsiedenden Amin - wie z.B. IPDA - zu iminieren, um dann, nach Abtrennung des Reaktionswassers, in mehrstufiger Fahrweise zuerst mit Ammoniak umzuiminieren und dann temperaturgestaffelt zu hydrieren.

Die Kombination aller in EP-A-394 967 vorgeschlagenen Maßnahmen führt zu einer max. Ausbeute von 90 % bei gleichzeitig niedriger Raum-Zeit-Ausbeute.

Der vorliegenden Erfindung lag daher die Aufgabe zugrunde, den zuvor genannten Nachteilen abzuhelfen.

Demgemäß wurde ein neues und verbessertes Verfahren zur Herstellung von 3-Aminomethyl-3,5,5-trimethylcyclohexylamin durch Umsetzung von 3-Cyano-3,5,5-trimethylcyclohexanon mit 3-Aminomethyl-3,5,5-trimethylcyclohexylamin und anschließender oder gleichzeitiger Zugabe von Ammoniak unter einem Wasserstoffdruck von 50 bis 300 bar in Gegenwart eines Hydrierkatalysators bei Temperaturen von 20 bis 150°C gefunden, welches dadurch gekennzeichnet ist, daß man die Umsetzung ohne Abtrennung des Reaktionswassers durchführt.

Das erfindungsgemäße Verfahren ist insofern bemerkenswert, als im Hinblick auf EP-A-394 967 nicht zu erwarten war, daß ohne Abtrennen des Reaktionswassers oder sonstiger das Iminierungsgleichgewicht positiv beeinflussender Faktoren und im wesentlichen ohne Zusatz eines Lösungsmittels Selektivitäten von bis zu 94 % zu erzielen sind. Bemerkenswert ist außerdem der minimale Anteil an hochsiedenden Komponenten, obwohl auf eine Verfahrensstufe der Umiminierung mit Ammoniak - wie in EP-A-394 967 ausgeführt - verzichtet wurde.

Das Verfahren läßt sich einstufig oder zweistufig [1. Stufe Kondensation von 3-Cyano-3,5,5-trimethylcyciohexanon mit 3-Aminomethyl-3,5,5-trimethylcyclohexylamin (Iminierung), 2. Stufe Umiminierende Hydrierung] an einem handelsüblichen Hydrierkatalysator ohne Abtrennung des Reaktionswassers und bevorzugt im wesentlichen in Abwesenheit eines zusätzlichen Lösungsmittels durchführen. Bevorzugt wird die einstufige Fahrweise.

Bei der einstufigen Fahrweise können entweder diskontinuierlich im Autoklaven oder Schlaufenreaktor an einem Suspensionskatalysator (10 bis 50 Gew.-%, bevorzugt 20 bis 40 Gew.-%, besonders bevorzugt 25 bis 35 Gew.-%) oder kontinuierlich im Rohrreaktor an einem Festbettkatalysator (bei Belastungen von 0,05 bis 0,5 kg/lh, bevorzugt 0,1 bis 0,2 kg/lh) bevorzugt diskontinuierlich im Autoklaven, 3-Cyano-3,5,5-trimethylcyclohexanon und 3-Aminomethyl-3,5,5-trimethylcyclohexylamin in einem Molverhältnis von 0,5 : 1 bis 5 : 1, bevorzugt 0,8 : 1 bis 2 : 1, besonders bevorzugt 1 : 1, bei 50 bis 150°C, bevorzugt 80 bis 130°C unter Zugabe von Ammoniak zum 3-Cyano-3,5,5-trimethylcyclohexanon im Gewichtsverhältnis von 0,1 : 1 bis 10 : 1, bevorzugt 0,2 : 1 bis 2 : 1, bei einem Wasserstoffdruck von 50 bis 300 bar, bevorzugt 100 bis 250 bar umgesetzt werden.

Die zweistufige Fahrweise kann entweder zeitlich getrennt in einem Autoklaven oder Schlaufenreaktor oder räumlich getrennt erfolgen, wobei die Kondensation katalysiert oder unkatalysiert, bevorzugt unkatalysiert, entweder kontinuierlich in einer Rührkaskade oder einem Rohrreaktor oder diskontinuierlich in einem Rührkessel durchgeführt wird und die anschließende Hydrierung kontinuierlich in einer Rührkesselkaskade (suspensionskatalysiert) oder einem Rohrreaktor (heterogenkatalysiert) oder diskontinuierlich in einem Rührkessel (suspensionskatalysiert) durchgeführt werden kann. Bevorzugt wird die zeitlich-zweistufige Fahrweise in einem Autoklaven oder Schlaufenreaktor. Hierbei werden 3-Cyano-3,5,5-trimethylcyclo-hexanon und 3-Aminomethyl-3,5,5-trimethylcyclohexylamin in einem Molverhältnis von 0,5 : 1 bis 5 : 1, bevorzugt 0,8 : 1 bis 2 : 1, besonders bevorzugt 1 : 1, bei 20 bis 100°C, bevorzugt 40 bis 60°C 0,5 bis 3 h, bevorzugt 1 bis 2 h gerührt und nach Zugabe des Suspensionskatalysators (10 bis 50 Gew.-%, bevorzugt 30 Gew.-%), und von 0,1 bis 10 kg Ammoniak pro kg 3-Cyano-3,5,5-trimethylcyclohexanon, bevorzugt 0,2 bis 2 kg/kg bei einem Wasserstoffdruck von 50 bis 300 bar, bevorzugt 100 bis 250 bar und einer Hydriertemperatur von 50 bis 150°C, bevorzugt 80 bis 130°C umgesetzt.

Als Hydrierkatalysatoren können in der Regel alle handelsüblichen Hydrierkatalysatoren eingesetzt werden, die eines oder mehrere der Elemente aus der Gruppe Kupfer oder Elemente der VIII. Nebengruppe des Periodensystems der Elemente, bevorzugt Nickel, Cobalt, Eisen, Kupfer und/oder Ruthenium enthalten. Je nach Einsatz verwendet man die Katalysatoren als Suspensions- oder Festbettkatalysatoren (Vollkontakt oder Trägerkatalysatoren). Bevorzugt verwendet man Raney-Nickel oder Raney-Cobalt als Suspensionskatalysator, Cobalt-Voll- oder Cobalt-Träger-Katalysatoren als Festbettkontakt. Als Träger eignen sich Siliciumdioxid, Aluminiumoxid, Titandioxid, Aktivkohle oder Zeolithe.

Das erfindungsgemäße Verfahren wird im wesentlichen in Abwesenheit eines (zusätzlihen) Lösungsmittels wie z.B. eines Alkohols und/oder eines Kohlenwasserstoffes durchgeführt. In der Regel beträgt der Anteil des zusätzlichen Lösungsmittels 0 bis 10 Gew.-%, bevorzugt 0 bis 5 Gew.-%, besonders bevorzugt wird kein zusätzliches Lösungsmittel (0 Gew.-%) eingesetzt.

3-Aminomethyl-3,5,5-trimethylcyclohexylamin wird als Ausgangsprodukt zur Herstellung von Isophorondiisocyanaten, als Aminkomponente für Polyamide und als Härter für Epoxidharze verwendet.

### Beispiele

### Beispiel 1

In einem Rührkolben wurden 165 g (1 mol) 3-Cyano-3,5,5-trimethylcyclohexanon zusammen mit 170 g (1 mol) 3-Aminomethyl-3,5,5-trimethylcyclohexylamin 2 h bei 50°C gerührt. Der schmelzeartige Austrag wurde dann in einem Rührautoklaven mit Begasungsrührer überführt und nach Zugabe von 100 g Raney-Cobalt und 560 ml (20 mol) flüssigem Ammoniak 10 h bei 100°C und 250 bar Wasserstoffdruck umgesetzt.

Der Hydrieraustrag enthielt:

| | |
|---|---|
| 3-Aminomethyl-3,5,5-trimethylcyclohexylamin | 97,0 Gew.-% |
| 1,3,3-Trimethyl-6-aza-bicyclo-(3,2,1)octan | 1,9 Gew.-% |
| 3-Aminomethyl-3,5,5-trimethylcyclohexanol | 0,2 Gew.-% |
| Spaltprodukte | 0,3 Gew.-% |

Die Ausbeute bezüglich 3-Cyano-3,5,5-trimethylcyclohexanon betrug 94,1 %.

### Beispiel 2

In einem Rührkolben wurden 165 g (1 mol) 3-Cyano-3,5,5-trimethylcyclohexanon zusammen mit 170 g (1 mol) 3-Aminomethyl-3,5,5-trimethylcyclohexylamin 2 h bei 50°C gerührt. Der schmelzeartige Austrag wurde dann in einem Rührautoklaven mit Begasungsrührer überführt und nach Zugabe von 100 g Raney-Cobalt und 560 ml (20 mol) flüssigem Ammoniak 10 h bei 130°C und 115 bar Wasserstoff umgesetzt.

Der Hydrieraustrag enthielt:

| | |
|---|---|
| 3-Aminomethyl-3,5,5-trimethylcyclohexylamin | 95,6 Gew.-% |
| 1,3,3-Trimethyl-6-aza-bicyclo-(3,2,1)octan | 2,5 Gew.-% |
| 3-Cyano-3,5,5-trimethylcyclo-hexylamin | 2,0 Gew.-% |
| Spaltprodukte | 0,4 Gew.-% |

Die Ausbeute bezüglich 3-Cyano-3,5,5-trimethylcyclohexanon betrug 91,2 %.

### Beispiel 3

In einem Rührkolben wurden 165 g (1 mol) 3-Cyano-3,5,5-trimethylcyclohexanon zusammen mit 170 g (1 mol) 3-Aminomethyl-3,5,5-trimethylcyclohexylamin 2 h bei 50°C gerührt. Der schmelzeartige Austrag wurde dann in einem Rührautoklaven mit Begasungsrührer überführt und nach Zugabe von 100 g Raney-Cobalt und 210 ml (7,4 mol) flüssigem Ammoniak 10 h bei 130°C und 150 bar Wasserstoff umgesetzt.

Der Hydrieraustrag enthielt:

| | |
|---|---|
| 3-Aminomethyl-3,5,5-trimethylcyclohexylamin | 95,8 Gew.-% |
| 1,3,3-Trimethyl-6-aza-bicyclo-(3,2,1)octan | 3,5 Gew.-% |
| 3-Aminomethyl-3,5,5-trimethylcyclohexanol | 0,3 Gew.-% |
| Spaltprodukte | 0,5 Gew.-% |

Die Ausbeute bezüglich 3-Cyano-3,5,5-trimethylcyclohexanon betrug 91,6 %.

### Beispiel 4

In einem Rührautoklaven mit Begasungsrührer wurden 165 g (1 mol) 3-Cyano-3,5,5-trimethylcyclohexanon zusammen mit 170 g (1 mol) 3-Amino-methyl-3,5,5-trimethylcyclohexylamin nach Zugabe von 100 g Raney-Cobalt und 560 ml (20 mol) flüssigem Ammoniak 10 h bei 100°C und 250 bar Wasserstoffdruck umgesetzt.

Der Hydrieraustrag enthielt:

| | |
|---|---|
| 3-Aminomethyl-3,5,5-trimethylcyclohexylamin | 96,6 Gew.-% |
| 1,3,3-Trimethyl-6-aza-bicyclo-(3,2,1)octan | 2,9 Gew.-% |
| 3-Cyano-3,5,5-trimethylcyclo-hexylamin | 0,05 Gew.-% |
| 3-Aminomethyl-3,5,5-trimethylcyclohexanol | 0,05 Gew.-% |
| Spaltprodukte | 0,3 Gew.-% |

Die Ausbeute bezüglich 3-Cyano-3,5,5-trimethylcyclohexanon betrug 93,1 %.

Die Analytik (Gew.-%) der Hydrierausträge erfolgte auf einer Kapillarsäule (25 m OV1, 80-15-280).

## Patentansprüche

1. Verfahren zur Herstellung von 3-Aminomethyl-3,5,5-trimethylcyclohexylamin durch Umsetzung von 3-Cyano-3,5,5-trimethylcyclohexanon mit 3-Aminomethyl-3,5,5-trimethylcyclohexylamin und anschließender oder gleichzeitiger Zugabe von Ammoniak unter einem Wasserstoffdruck von 50 bis 300 bar in Gegenwart eines Hydrierkatalysators bei Temperaturen von 20 bis 150°C, dadurch gekennzeichnet, daß man die Umsetzung ohne Abtrennung des Reaktionswassers durchführt.

2. Verfahren zur Herstellung von 3-Aminomethyl-3,5,5-trimethylcyclohexylamin nach Anspruch 1, dadurch gekennzeichnet, daß man 3-Cyano-3,5,5-trimethylcyclohexanon mit 3-Aminomethyl-3,5,5-trimethylcyclohexylamin im Molverhältnis von 0,5 : 1 bis 5 : 1 einsetzt.

3. Verfahren zur Herstellung von 3-Aminomethyl-3,5,5-trimethylcyclohexylamin nach Anspruch 1, dadurch gekennzeichnet, daß man Ammoniak und 3-Cyano-3,5,5-trimethylcyclohexanon im Gewichtsverhältnis von 0,1 : 1 bis 10 1 einsetzt.

4. Verfahren zur Herstellung von 3-Aminomethyl-3,5,5-trimethylcyclohexylamin nach Anspruch 1, dadurch gekenzeichnet, daß man Hydrierkatalysatoren einsetzt, die mindestens eines der Elemente ausgewählt aus der Gruppe von Kupfer oder ein Element der VIII. Nebengruppe des Periodensystems der Elemente enthalten.

5. Verfahren zur Herstellung von 3-Aminomethyl-3,5,5-trimethylcyclohexylamin nach Anspruch 1, dadurch gekenzeichnet, daß man als Hydrierkatalysatoren Raney-Cobalt oder Raney-Nickel einsetzt.

6. Verfahren zur Herstellung von 3-Aminomethyl-3,5,5-trimethylcyclohexylamin nach Anspruch 1, dadurch gekenzeichnet, daß man als Hydrierkatalysatoren Cobalt-Voll- oder Cobalt-Träger-Katalysatoren einsetzt.

7. Verfahren zur Herstellung von 3-Aminomethyl-3,5,5-trimethylcyclohexylamin nach Anspruch 1, dadurch gekenzeichnet, daß man im wesentlichen in Abwesenheit eines Lösungsmittels arbeitet.

## Claims

1. A process for the preparation of 3-aminomethyl-3,5,5-trimethylcyclohexylamine by reacting 3-cyano-3,5,5-trimethylcyclohexanone with 3-aminomethyl-3,5,5-trimethylcyclohexylamine and subsequently or simultaneously adding ammonia under a hydrogen pressure of from 50 to 300 bar in the presence of a hydrogenation catalyst at from 20 to 150°C, which comprises carrying out the reaction without removing the water of reaction.

2. A process for the preparation of 3-aminomethyl-3,5,5-trimethylcyclohexylamine as claimed in claim 1, wherein 3-cyano-3,5,5-trimethylcyclohexanone and 3-aminomethyl-3,5,5-trimethylcyclohexylamine are used in a molar ratio of from 0.5 : 1 to 5 : 1.

3. A process for the preparation of 3-aminomethyl-3,5,5-trimethylcyclohexylamine as claimed in claim 1, wherein ammonia and 3-cyano-3,5,5-trimethylcyclohexanone are used in a weight ratio of from 0.1 : 1 to 10 : 1.

4. A process for the preparation of 3-aminomethyl-3,5,5-trimethylcyclohexylamine as claimed in claim 1, wherein a hydrogenation catalyst which contains at least one of the elements selected from the group consisting of copper or an element of subgroup VIII of the Periodic Table of Elements is used.

5. A process for the preparation of 3-aminomethyl-3,5,5-trimethylcyclohexylamine as claimed in claim 1, wherein the hydrogenation catalyst used is Raney cobalt or Raney nickel.

6. A process for the preparation of 3-aminomethyl-3,5,5-trimethylcyclohexylamine as claimed in claim 1, wherein the hydrogenation catalyst used is an unsupported or supported cobalt catalyst.

7. A process for the preparation of 3-aminomethyl-3,5,5-trimethylcyclohexylamine as claimed in claim 1, wherein the reaction is carried out essentially in the absence of a solvent.

## Revendications

1. Procédé de préparation de 3-aminométhyl-3,5,5-triméthylcyclohexylamine par réaction de 3-cyano-3,5,5-triméthylcyclohexanone avec la 3-aminométhyl-3,5,5-triméthylcyclohexylamine et addition subséquente ou simultanée d'ammoniac sous une pression d'hydrogène de 50 à 300 bar en présence d'un catalyseur d'hydrogénation à des températures de 20 à 150°C, caractérisé en ce que l'on effectue la réaction sans séparation de l'eau de la réaction.

2. Procédé de préparation de 3-aminométhyl-3,5,5-triméthylcyclohexylamine selon la revendication 1, caractérisé en ce que l'on utilise la 3-cyano-3,5,5-triméthylcyclohexanone avec la 3-aminométhyl-3,5,5-triméthylcyclohexylamine dans un rapport en moles de 0,5 : 1 à 5 : 1.

3. Procédé de préparation de 3-aminométhyl-3,5,5-triméthylcyclohexylamine selon la revendication 1, caractérisé en ce qu'on utilise l'ammoniac et la 3-cyano-3,5,5-triméthylcyclohexanone dans un rapport en poids de 0,1 : 1 à 10 : 1.

4. Procédé de préparation de 3-aminométhyl-3,5,5-triméthylcyclohexylamine selon la revendication 1, caractérisé en ce qu'on utilise des catalyseurs d'hydrogénation qui contiennent au moins un élément choisi dans le groupe du cuivre ou un élément du groupe VIII de la classification périodique des éléments.

5. Procédé de préparation de 3-aminométhyl-3,5,5-triméthylcyclohexylamine selon la revendication 1, caractérisé en ce qu'on utilise comme catalyseurs d'hydrogénation du cobalt de Raney ou du nickel de Raney.

6. Procédé de préparation de 3-aminométhyl-3,5,5-triméthylcyclohexylamine selon la revendication 1, caractérisé en ce qu'on utilise comme catalyseurs d'hydrogénation des catalyseurs supportés au cobalt ou des catalyseurs massiques au cobalt.

7. Procédé de préparation de 3-aminométhyl-3,5,5-triméthylcyclohexylamine selon la revendication 1, caractérisé en ce que l'on travaille pour l'essentiel en l'absence de solvant.
